(19) Europäisches Patentamt / European Patent Office / Office européen des brevets

(11) **EP 2 786 768 A1**

(12) **EUROPEAN PATENT APPLICATION**

(43) Date of publication:
**08.10.2014 Bulletin 2014/41**

(51) Int Cl.:
*A61K 49/18* (2006.01)  *A61K 49/10* (2006.01)

(21) Application number: **13162339.9**

(22) Date of filing: **04.04.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Agfa Healthcare**
**2640 Mortsel (BE)**

(72) Inventors:
• **Buffel, Diederik**
  **2640 Mortsel (BE)**
• **Leblans, Paul**
  **2640 Mortsel (BE)**
• **Venneman, Jan**
  **2640 Mortsel (BE)**

(54) **Process for preparing a material comprising a macrocyclic ligand and for producing a pharmaceutical formulation comprising said ligand with a lanthanide**

(57)    The present invention relates to a process for preparing a macrocyclic ligand and for producing a pharmaceutical liquid formulation comprising a complex of said ligand with a lanthanide or similar compounds.

The macrocyclic ligand of the present invention is a derivative of tetraaza macrocycles such as 1,4,7,10-tetraazacyclododecane (cyclen), 1,4,7,10-tetrazacyclotridecan (homocyclen) and 1,4,8,11-tetraazacyclotetradecane (cyclam), such as DOTA, TRITA, TETA, DOTMA, TCE-DOTA, DOTA-pNB, D03A, HP-D03A, D03A-butrol, D03MA, ODOTRA, D03A-L2, DOTP, DOTMP, DO2a, THP, THED, DOTAM, DOTTA. The preferred lanthanide and similar compounds are Gadolinium (Gd), Yttrium (Y) and Terbium (Tb).

The process of the present invention aims to obtain an accurate balance between the ligand and the lanthanide, and to avoid the presence of free lanthanide in said formulation, by calculating the necessary amount of ligand for a formulation batch, measuring the moisture content of a sample of the material in said batch, calculating the total amount of moisture present of the batch and calculating the total amount of material which is required to prepare the batch size.

In this way, the production of a pharmaceutical liquid formulation comprising a complex of a macrocyclic ligand with lanthanide is more accurate, faster and easier. The present invention is thus useful for the production of pharmaceutical liquid formulations comprising a complex of a macrocyclic ligand with a lanthanide, which can be used as contrast agents for magnetic resonance imaging.

EP 2 786 768 A1

**Description**

**FIELD OF THE INVENTION**

The present invention relates to a process for preparing a material comprising a macrocyclic ligand and for producing a pharmaceutical liquid formulation of a complex of said ligand with a lanthanide or a similar compound, which can be used as contrast agents for magnetic resonance imaging.

**BACKGROUND OF THE INVENTION**

Magnetic resonance imaging (MRI) is a powerful, non-invasive technique used to produce detailed two or three-dimensional anatomical images of tissues in the body. Conventional MRI uses the proton $^1$H as its signal source which is highly abundant in tissues and it has the highest sensitivity of all the biologically relevant nuclei.

The contrast, which makes the differentiation of internal structures possible in the image, arises from how the signal decays and is the difference between the resulting signals from two tissue regions. The route by which the protons release the energy they absorbed from the radio-frequency pulse, thus reducing the transverse magnetisation and causing signal decay, is known as relaxation. In MRI two independent relaxation processes occur simultaneously: spin-lattice or longitudinal relaxation characterised by the time constant $T_1$, and spin-spin or transverse relaxation, characterised by the time constant $T_2$.

Often, when suitable $T_1$- or $T_2$-weighting sequences are used, the natural contrast between two tissues is enough to produce a diagnostically-useful image. However, some conditions do not lead to specific enough changes in the relaxation times of the affected tissue though and then a contrast agent is used to locally change the relaxation times of the diseased tissue, improving the image contrast.

Most contrast agents work by shortening the relaxation times of the water protons in the targeted tissue. $T_1$ contrast agents are based on paramagnetic metal ion chelates which make the tissue appear brighter on the $T_1$-weighted image (positive contrast). $T_2$ contrast agents are usually superparamagnetic iron oxide nanoparticles which create dark spots on the $T_2$-weighted image (negative contrast). $T_1$ agents are the most widely used and the majority of these are based on chelates of the gadolinium ion ($Gd^{3+}$).

To be an effective $T_1$ agent the lanthanide chelate must significantly increase the proton relaxation rates in water. Lanthanide elements are most commonly found in the +3 oxidation state ($Ln^{+3}$), corresponding to the electronic configuration $[Xe]6s^2 4f^n$. Gadolinium (Gd)is the seventh element in the lanthanide series and has an electronic configuration $[Xe]4f^7$. This means that $Gd^{3+}$ has seven unpaired electrons, making it highly paramagnetic i.e. Gd(III) ions have large permanent magnetic moments (due to electron spin angular momentum), but in the absence of an external magnetic field these are randomly oriented. Due to its large size the Gd(III) and other lanthanides ions typically have a coordination number of nine in its complexes. As a free ion lanthanides, and in particular gadolinium, are very toxic for the tissues but it is generally considered safe when said ions are administrated as a chelated compound.

The level of toxicity depends on the strength of the chelating agent, also known as ligand, chelator or sequestering agent. Usually these ligands are organic compounds which form two or more separate coordinate bonds with a single central metal ion, in this case, the gadolinium ion, inactivating it thus reducing or eliminating its toxic effect in the tissues.

Polyaminopolycarboxylic acid compounds are the ligand type of choice because they form exceptionally stable complexes with the Gd(III) ion, which can be explained by a number of reasons. These compounds can be linear (such as pentetic acid or diethylene triamine pentaacetic acid also named as DTPA) or macrocyclic (such as 1,4,7,10-tetraazacyclododecane-1,4,7,10-tetraacetic acid, DOTA). Complexes of macrocyclic ligands are much more kinetically inert and thus, present an exceptionally high solution stability.

Several contrast agents comprising gadolinium as the lanthanide are marketed, Magnevist® (Bayer Healthcare) with gadopentetate dimeglumine, Multihance® (Bracco) with gadobenate dimeglumine, Omniscan® (GE Healthcare) with gadodiamide, Optimark® (Mallinckrodt Inc.) with gadoversetamide and the macrocyclic chelates Dotarem® (Guerbet) with gadoteric acid, Prohance® (Bracco) with gadoteridol and Gadovist® (Bayer Healthcare) with gadobutrol.

In the body, the complexes of chelates with lanthanide are in a situation of chemical equilibrium, which may lead to a risk of undesired release of the lanthanide, and more especially of gadolinium. Release of $Gd^{3+}$ from the complex is said to be responsible for the toxicity associated with gadolinium complexes; this release appears to be a consequence of $Zn^{2+}$, $Cu^{2+}$, and $Ca^{2+}$ transmetallation *in vivo.*

A person skilled in the art is thus led to seek technical solutions that limit this risk in order to solve the complex problem of tolerance in the patient as safely as possible. This problem is all the more difficult since the administration of contrast agents is often repeated during diagnostic examinations and/or for the guiding and monitoring of the efficacy of a therapeutic treatment.

Many contrast agents based on macrocyclic complexes of chelates with gadolinium are known and are described, for example, in documents EP0270483, US4647447, WO2010130814 and WO2009103744.

EP0270483 discloses contrast agents based on gadolinium with addition of one or more free ligands, such DOTA (1,4,7,10-tetraazacyclododecane-1,9,7,10-tetraacetic acid), EDTA (ethylenediaminetetraacetic acid) or DTPA (diethylene triamine pentaacetic acid) and/or one or more weak metal complexes (presenting relatively low stability constant, such as calcium, magnesium, zinc and iron) or their mixtures to pharmaceutical agents based on metal complexes result in contrast agents with improved in vivo tolerance. These contrast agents are prepared by addition of a certain amount of the free ligand to the metal complex in aqueous medium, which is further evaporated to dryness in vacuum. However, there is always a difference in the amount of components in the calculated formulation and in the obtained formulation due to the presence of a variable amount of water in the ligand, which generates an error in the exact amount of ligand that is introduced in the formulation. This variation in water content can be important for certain ligands, such as for DOTA (1,4,7,10-tetraazacyclododecane-1,4,7,10-tetraacetic acid), TRITA (1,4,7,10-Tetraazacyclotridecane-1,4,7,10-tetraacetic acid), TETA (1,4,8,11-Tetraazacyclotetradecane-1,4,8,11-tetraacetic acid), HP_D03A (1,4,7,10-Tetraazacyclododecane-1,4,7-triacetic acid, 10-(2-hydroxypropyl)), D03A-butrol (1,4,7,10-Tetraazacyclododecane-1,4,7-triacetic acid, 10-[2,3-dihydroxy-1-(hydroxymethyl)propyl]) and depends on the conditions of temperature and relative humidity during drying and filling of said ligands. In powder form they can contain between 0% and more than 10% water and the water content can change afterwards during contact with the atmosphere. This change will be greater when the relative humidity conditions during the weighing and filling step of the formulation are different from those during drying and filling of the ligand itself. When the relative humidity during weighing for the formulation step is higher than during the drying and filling step, the weight of the powder will have increased because of water uptake from the atmosphere thus causing an error in the exact weight of the ligand and thus in the composition of the formulated contrast agent.

US4647447 discloses complex salts from the anion of a complexing acid and one or more central ions of an element with an atomic number of 21 to 29, 42, 44 or 57 to 83 and, optionally, also formed from one or more physiologically biocompatible cations of an inorganic and/or organic base or amino acid. Said complex salts may be also coupled as conjugates with biomolecules known to concentrate in a specific organ to produce more efficient contrast agents. The contrast agents herein disclosed are produced by suspension or dissolution of said complex salts in an aqueous medium, with or without additives, followed by sterilization. This document also discloses the production of contrast agents without isolating the respective complex salts. In this case, to avoid the presence of free toxically active metal ions, such as $Ga^{3+}$, the chelating step is done by using colour indicators such as xylenol orange by control titrations. According to US4647447, the contrast agents obtained show great stability in vitro and in vivo and are exceptionally well tolerated physiologically. However, the reliability of this process depends largely on the type of chelate and lanthanide. In the case of highly hygroscopic chelates it is not possible to assure that the correct balance between both is present in the body, in particular when other ions are present, such $Ca^{2+}$, $Zn^{+2}$ and $Cu^{2+}$ which are able to replace the lanthanide ion in some extent, in the complex and thus releasing these to the body.

WO2010130814 discloses a process for preparing contrast agents in powder form based on a lanthanide chelate, said powder including an excess of free chelate of 0.002% to 0.4% mol/mol to address the problem of in vivo intolerance of lanthanide chelates related to the presence of free lanthanide ions in the formulation to be administered. Said process includes a step a) of mixing the chelate and the lanthanide wherein the free chelate is in excess in relation to the amount of the lanthanide; b) measuring the amount of free chelate and adjusting said amount to an excess of 0.002 to 0.4% mol/mol in relation to the amount of the lanthanide; and c) precipitating the complexing solution obtained with or without the adjustment step in an organic solvent, thus obtaining a powder of chelate-lanthanide, wherein said powder contains an amount of free chelate in excess in relation to the amount of the lanthanide. Finally, excipients and water are added to produce an injectable contrast agent composition. Once again, it is not possible to assure that the correct balance between chelate and lanthanide is present in the final formulation to be administered in particular in what regards when linear chelates are used mainly due to their fast dissociation kinetics. Moreover, another disadvantage of this process is that several steps are performed and adjusting the amount of chelate in excess, in powder form, is not an easy and reliable way to obtain the intended amount due to hygroscopic properties of these compounds.

WO2009103744 discloses a process to produce a liquid formulation comprising a complex of a macrocyclic chelate with a lanthanide, having an excess of free chelate of between 0.02-0.4% and comprising the steps of a) preparing a liquid formulation of said macrocyclic chelate with the lanthanide; b) measuring the amount of the macrocyclic chelate in the formulation; c) adjusting the amount of macrocyclic chelate in order to obtain an excess of free chelate between 0.02-0.4% in relation to the amount of free lanthanide and that there is no free lanthanide present in the final formulation. However, all these steps increase the risk of occurring errors and of introducing microbiological contaminants. Furthermore, it also makes the process expensive and time consuming.

It is thus desirable to obtain an optimized process for producing a pharmaceutical liquid formulation comprising a complex of macrocyclic ligand with a lanthanide, which can be used as contrast agent for magnetic resonance imaging with good tolerance in vivo. Furthermore, it is also desirable to simplify the method for producing contrast agents formulations at an industrial scale.

## SUMMARY OF THE INVENTION

It is an object of the present invention to provide a process for for preparing a macrocyclic ligand that allows measuring the amounts of said ligand in a fast and accurate way.

It is another object of the present invention to provide a process for producing a pharmaceutical liquid formulation comprising a complex of macrocyclic ligand with a lanthanide or a similar compound that allows obtaining safe contrast agents in a simple, straightforward and reliable process.

This object is realised by providing a process for preparing a material comprising a macrocyclic ligand as defined in claim 1.

It is also provided a process for producing a complex material of a macrocyclic ligand with a lanthanide or a similar compound as defined in claim 4.

Another object of the present invention is to provide a pharmaceutical liquid formulation that can be used as a contrast agent comprising a macrocyclic ligand material prepared according to the present invention, as defined in claim 7.

Further advantages and embodiments of the present invention will become apparent from the following description and the dependent claims.

## DESCRIPTION OF THE INVENTION

The present invention relates to a process for preparing a macrocyclic ligand material and to a process for producing a pharmaceutical liquid formulation comprising a complex of macrocyclic ligand with a lanthanide or a similar compound wherein said ligand is prepared according to the present invention, in a fast and reliable way, by controlling the exact weight of the macrocyclic ligand in the final formulation and encompasses the following steps:

- calculating the necessary amount of a material comprising a macrocyclic ligand for a formulation batch;
- measuring the moisture content of said material in a sample of the batch;
- calculating the total amount of moisture present in said batch and
- calculating the total amount of material which is required for preparing the batch size.

The pharmaceutical liquid formulation is then produced by adding the remaining components of the formulation, such as a lanthanide or a similar compound and excipients such as meglumine, to the material comprising the macrocyclic ligand.

In one embodiment, the total amount of the material comprising a macrocyclic ligand can be divided into a plurality of portions and distributed into unit packages under controlled conditions of humidity.

In another embodiment, when a certain amount (Y) of material comprising a macrocyclic ligand is taken as a sample for controlling purposes, ex. quality control, then said amount (Y) may be added to the calculated amount of material in order to compensate the amount that was taken for testing.

In the scope of the present invention the ligand is preferably a derivative of tetraaza macrocycles such as 1,4,7,10-tetraazacyclododecane (cyclen), 1,4,7,10-tetrazacyclotridecan (homocyclen) and 1,4,8,11-tetraazacyclotetradecane (cyclam), preferably DOTA (1,4,7,10-tetraazacyclododecane-1,4,7,10-tetraacetic acid), NOTA (1H-1,4,7-Triazonine-1,4,7-triacetic acid, hexahydro), DOTAGA (1,4,7,10-Tetraazacyclododecane-1,4,7,10-tetraacetic acid, $\alpha$-(2-carboxyethyl)), D03A (1,4,7,10-Tetraazacyclododecane-1,4,7-triacetic acid), D03A-butrol (1,4,7,10-Tetraazacyclododecane-1,4,7-triacetic acid, 10-[2,3-dihydroxy-1-(hydroxymethyl)propyl]), HP-D03A (1,4,7,10-Tetraazacyclododecane-1,4,7-triacetic acid, 10-(2-hydroxypropyl)) and PCTA (3,6,9,15-Tetraazabicyclo[9.3.1]pentadeca-1(15),11,13-triene-3,6,9-triacetic acid), more preferably DOTA, D03A, HP-D03A and even more preferably DOTA.

In the scope of the present invention, "lanthanides" comprise the fifteen metallic chemical elements with atomic numbers 57 through 71, from lanthanum through lutetium. "Similar compounds" comprise scandium and yttrium. Together with scandium and yttrium, the trivial name "rare earths" is sometimes used to describe all the lanthanides and similar compunds.

Preferred lanthanides and similar are Gadolinium (Gd), Yttrium (Y) and Terbium (Tb) and most preferred is Gadolinium.

### Description of a preferred embodiment of the invention

### I - Process for preparing the macrocyclic ligand

In a first step it is necessary to calculate the necessary amount of material comprising a macrocyclic ligand for producing a batch of a certain size to obtain the desired contrast agent formulation. This material comprises active macrocyclic ligand which may have moisture.

**1. Calculating the necessary amount of macrocyclic ligand**

The total amount of macrocyclic ligand (X1) for a specific batch must be calculated in a way that it is present in the final formulation in an excess (Lf) in relation of the total amount of lanthanide or a similar compound (G), being said amount (Lf) in the range from 0.002% to 0.4% in relation of the amount of lanthanide or similar compound, preferably in the range from 0.02% to 0.3%, more preferably in the range from 0.025% to 0.25%, meaning that a small amount of excess ligand is added to the formulation. This may ensure that metal traces originated during its production and/or sterilization can be trapped, thus avoiding any possibility of replacement the lanthanide or similar compound ions in the complex that may result in their release in free form in the contrast agent formulation.

This means that in the final formulation there is an amount of macrocyclic ligand forming a complex with lanthanide or similar compound (LG) and an amount in free form (Lf), not complexing the lanthanide or similar compound ions and thus:

$$X1 = LG + Lf$$

Wherein:

. X1 is the total amount of macrocyclic ligand present in the final contrast agent formulation, i.e. the target amount of ligand able to form a complex;
. LG is the amount of macrocyclic ligand forming a complex with the lanthanide or similar compound present in the final contrast agent formulation; and
. Lf is the amount of macrocyclic ligand in its free form present in the final contrast agent formulation, which according to the present invention is in the range set out above in relation of the total amount of the lanthanide or similar compound(G),

The size of the batch can be small, suitable for laboratory scale i.e. for example of 250g, 500g 1000g, etc. or bigger sizes such as 20kg, 50kg, 200kg, 500kg, etc. for pilot and industrial scale.

The total amount of macrocyclic ligand (X1) can be produced by any known method in the art and is homogenised in a container. Said container may be the container where the reaction for producing the ligand occurred, a blender, a homogeniser or any container suitable for homogenising the obtained macrocyclic ligand in the desired amount for a certain batch of formulation.

**2. Measuring the moisture content of the material comprising a macrocyclic ligand**

In this step, a sample of the homogenised material comprising the macrocyclic ligand to be used in the batch in question is taken for analysis from the container in order to determine the moisture content (M) of said batch. Based on this result, it is possible to calculate the actual amount in weight of ligand with moisture (X3). Thus,

$$X3 = X1 + M$$

wherein,

- X3 is the target weight of material comprising the macrocyclic ligand + moisture for the final formulation batch;
- X1 is the target weight of macrocyclic ligand excluding the moisture weight; and
- M is the weight of the moisture present in the material for the final formulation batch.

Now it is possible to further calculate the amount of material comprising the macrocyclic ligand necessary to fill each unit package when the batch is optionally divided into portions. Thus,

$$X2 = X3 / N1$$

Wherein,

X2 is the amount (X3) in weight of material comprising the macrocyclic ligand with moisture (M) in each unit package; and (N1) is the number of unit packages for one final formulation batch.

If the amount of material comprising the macrocyclic ligand + moisture is introduced into the formulation, the amount of ligand available to form the complex with a lanthanide or similar compound may be not enough when the amount of moisture (M) is not taken into account. Therefore, by measuring M it is possible to know the exact amount of ligand necessary to add to obtain a certain batch in order to avoid the presence of free lanthanide.

In the embodiment where the material comprising a macrocyclic ligand is divided into portions, each unit package is then filled with a homogenised and specifically measured amount (X2) of material under controlled conditions of humidity so that the powder ligand does not further absorb or desorb water during this phase. In consequence, the real content of macrocyclic ligand able to form a complex with a lanthanide or similar compound and of moisture in each unit package is known and thus no further measurements or adjustments are needed; when it is desired to use a certain amount of ligand to produce a batch of formulation it is only necessary to calculate the number of unit packages to achieve the total amount of necessary ligand.

The total amount (X2) of the material of the batch can be divided into portions and then packed. In such case, the amount (N1) of unit packages necessary to pack the material comprising the macrocyclic ligand can be also calculated in function of the total volume of the batch and the size of each unit package.

In another embodiment, the amount (X2) of material in each unit package takes into account the removal of a certain quantity (Y) of said material for testing purposes, ex. for quality control, certification purposes, etc. In this case, the amount (Y) of material to be removed for testing must be calculated and added to the total amount of material (X2) filled in each unit package to ensure that the total amount of macrocyclic ligand calculated previously is maintained and available to form a complex.

Therefore, if a sample of, for example, 1g is needed to test the material comprising the ligand for a determined purpose, such as certification, then the amount of material (X2) in each unit package must be corrected by addition of 1g to X2 to result in the exact amount of ligand necessary to produce a desired batch size, without the need of further measurements or adjustments.

### II - Process for producing a pharmaceutical liquid formulation

The remaining components necessary to obtain the liquid formulation comprising the macrocyclic ligand, prepared as described above, are added to the material prepared as described above or optionally to a certain number of unit packages in the desired amounts in function of the different contrast agent formulations. Therefore, this phase includes: a) the complex formation by addition of a selected lanthanide or similar compound to the selected amount of material comprising a macrocyclic ligand, and b) the addition of the necessary excipients to obtain a formulation with the desired pharmaceutical properties. Examples of such excipients are water, meglumine, hydrochloric acid and/or sodium hydroxide for pH adjustment.

### 3. Complex formation

The selected lanthanide or similar compound is added to the material comprising the ligand to form the complex macrocyclic ligand with metal ion in the form of oxide or salts thereof. The total amount of lanthanide or similar compound added to form the said complex depends on the amount of ligand desired to form a complex (LG), i.e. G should correspond to LG. The preferred lanthanide or similar compound is gadolinium, terbium or yttrium, most preferable compound is gadolinium. The preferred form of providing gadolinium is in the form of oxide, i.e. $Gd_2O_3$.

For this purpose, a mixture of material comprising the ligand and the lanthanide or similar compound is dissolved in the exact desired amount in a reaction vessel. No further measurements or adjustments are needed to produce a macrocyclic complex with a lanthanide or similar compound.

The amount of lanthanide or similar compound (Gf) added must be calculated in order to have no free lanthanide or similar compound in the final formulation, i.e. Gf = 0 in order to avoid toxicity problems associated with the administration of the contrast agent in cause.

### 4. Adding the remaining components or excipients

The remaining excipients are added also to the reaction vessel containing the complex of a macrocyclic ligand and the selected lanthanide or similar compound produced as described above.

The macrocyclic ligand is present in the final liquid formulation in a concentration in the range between 0.3 to 0.7 M, preferably in the range between 0.4 to 0.6 M and most preferably at 0.5 M.

The intended amount of free ligand (Lf) in the liquid formulation is in the range from 0.002% to 0.4 mol% in relation of the total amount of lanthanide or similar compound (G), preferably is in the range from 0.02% to 0.3 mol%, more preferably in the range from 0.025% to 0.25 mol% in relation to the total amount of lanthanide or similar compound (G).

Examples of excipients added to the complex of a macrocyclic ligand with a lanthanide or similar compound to obtain a liquid formulation as described above are water, an organic base, hydrochloric acid and/or sodium hydroxide for pH adjustment. pH is preferably in a range from 4 to 8.5 and more preferably in a range from 6.5 to 7.9.

## Measurements

The measurement of the content of water in DOTA is performed as described in the semi micro method of Karl Fischer (Fischer, Karl - Pharm. Eur. 2.5.12)

## EXAMPLES

All reagents used in the following examples were readily available from commercial sources unless otherwise specified.

All reagents used to prepare DOTA and gadoterate meglumine, including 1,4,7,10-tetraazacyclododecane (cyclen), $Gd_2O_3$ and N-methyl-D-glucamine, were obtained commercially and used as received.

## Example 1 - Preparation of a 20 kg batch of macrocyclic ligand DOTA

This example illustrates the calculation for preparing an amount of macrocyclic ligand for a batch size of liquid formulation of 200L. The selected ligand was DOTA and the lanthanide or similar compound was Gadolinium. The concentration of gadoterate complex in said formulation was 0.5M (M/L).

The total amount of DOTA (X1) was calculated in a way that it is present in the final formulation in an excess (Lf) in relation of the total amount of lanthanide (G), being said amount (Lf) in the range from 0.002% to 0.4% in relation of the amount of gadolinium, preferably in the range from 0.02% to 0.3%, more preferably in the range from 0.025% to 0.25%. A small amount of excess DOTA was then added to the formulation to accomplish this target. This means that in the final formulation there was an amount of DOTA forming a complex with gadolinium (LG) and an amount in free form (Lf), not complexed with the gadolinium ions and thus:

$$X1 = LG + Lf$$

Wherein

- X1 represents the total amount of DOTA present in the final contrast agent formulation;
- LG represents the amount of DOTA forming a complex with the gadolinium present in the final contrast agent formulation; and
- Lf represents the amount of DOTA in its free form present in the final contrast agent formulation, which according to the present invention is in the range set out above in relation of the total amount of the gadolinium (G),

Therefore, for a batch of 200 L of liquid formulation at 0.5M:

X1 = 100.002 to 100.4 moles of DOTA; this corresponds to 40.443 to 40.603 kg of DOTA 100%;
LG = 100 moles of complexed DOTA with gadolinium;
Lf = 0.002 to 0.4 moles of free DOTA, to achieve 0.002% to 0.4% moles/moles in relation of the amount of complexed gadolinium; assume a target Lf of 0.2% is calculated to minimize the risk of obtaining a preparation that does not meet the requirements of Lf between 0.002% and 0.4%. Therefore X1 should be 100.2 moles or 40.523 kg of DOTA;
G = 100 moles of complexed gadolinium; this corresponds to 50 moles of gadolinium oxide ($Gd_2O_3$).

Assuming that the moisture content of the said batch of DOTA is 5.00%, the extra weight of the moisture, M, is 2.026 kg and the total weight of DOTA + moisture, X2, is 42.549 kg, to be divided in an integer number of unit packages N. If the number of unit packages of N = 9 is chosen, then the weight of each unit package, X3, equals 4.728 kg.

## Example 2 - Measuring the moisture content of the ligand

This and the next example illustrate how the moisture content in a batch of a macrocyclic ligand changes when the product is exposed to different conditions of relative humidity.

The moisture content in the samples was measured using methods known to those skilled in the art. In this example, the measurement of the content of water in DOTA was performed as described in the semi micro method of Karl Fischer (Fischer, Karl - Pharm. Eur. 2.5.12).

Two samples of DOTA were kept in conditions of relative humidity (RH) = 30±5% at a temperature of Temp = 20±2°C. From these samples, small samples ware taken at different times (T0 = beginning of the experiment; T30 = T0 + 30 min; T90 = T0 + 90 min; and T300 = T0 + 300 min) and the water content was measured as described above. Each experiment (Exp.) was performed twice and the average of each (Ind.) was determined (Avg.). The results in percentage of the initial weight of DOTA are shown in Table 1.

Table 1 - Determination of water content of DOTA at Temp. = 20±2°C and RH = 30±5%

| | Water content DOTA (%) Temp. = 20±2°C; RH = 30±5% | | | | | | | |
|---|---|---|---|---|---|---|---|---|
| | $T_0$ | | $T_{30}$ | | $T_{90}$ | | $T_{300}$ | |
| | Ind. | Avg. | Ind. | Avg. | Ind. | Avg. | Ind. | Avg. |
| Exp.1 | 4.941 | 4.94 | 4.944 | 4.98 | 5.038 | 5.00 | 5.178 | 5.15 |
| | 4.929 | | 5.020 | | 4.954 | | 5.114 | |
| Exp.2 | 5.346 | 5.25 | 6.154 | 6.20 | 6.062 | 6.12 | 6.106 | 6.19 |
| | 5.150 | | 6.247 | | 6.170 | | 6.268 | |

These results show that at 30% RH the amount of moisture M in the batch increases, thus increasing the weight of DOTA powder (X2).

**Example 3 - Measuring the moisture content of the ligand in conditions of higher RH**

This example, as the previous one, illustrates how the moisture content in a batch of a macrocyclic ligand is affected by the environmental relative humidity conditions. Therefore, the experiments of Example 2 were repeated in the same conditions but with a RH = 75±5% instead of RH = 30±5% of Ex. 1. Again, each experiment (Exp.) was performed twice and the average of each (Ind.) was determined (Avg.). The results in percentage of the initial weight of DOTA are shown in Table 2.

Table 2 - Determination of water content of DOTA at Temp. = 20±2°C and RH= 75±5%

| | Water content DOTA (%) Temp.= 20±2°C; RH = 75±5% | | | | | | | |
|---|---|---|---|---|---|---|---|---|
| | $T_0$ | | $T_{30}$ | | $T_{90}$ | | $T_{300}$ | |
| | Ind. | Avg. | Ind. | Avg. | Ind. | Avg. | Ind. | Avg. |
| Exp.3 | 4.941 | 4.94 | 6.734 | 6.71 | 7.475 | 7.34 | 5.914 | 5.86 |
| | 4.929 | | 6.694 | | 7.200 | | 5.813 | |
| Exp.4 | 5.346 | 5.25 | 6.449 | 6.65 | 6.962 | 6.88 | 4.111 | 4.36 |
| | 5.150 | | 6.851 | | 6.789 | | 4.609 | |

These results show that the water content in DOTA increases with the time and with the relative humidity of the conditions and thus the high hygroscopicity of DOTA.

**Example 4 - Preparation of a complex of DOTA with Gadolinium**

In this example, the necessary amount of material comprising DOTA was divided into portions and packed into several unit packages. Using the amounts mentioned in the first Example, i.e. for a batch of liquid formulation of 200L, a Lf of 0.2% and for a total amount of DOTA (X1) of 40.523 kg and a moisture content M of 5.00%, then the total amount of DOTA + M (X2) equals 42.549 kg, to be divided in an integer number of packages, e.g. 9 packages of 4.785 kg. This integer number of packages has to be fed in the formulation reactor as such (no adjustment in the actual weight at the time of filling the reactor is required).

The weight of gadolinium oxide for this batch should then be of 100 equivalents, this is 50 moles or 18.125 kg of gadolinium oxide ($Gd_2O_3$) and thus, the meglumine amount to be added is 100 moles, 19.521 kg.

**Claims**

1. A process for preparing a batch of a material which comprises a macrocyclic ligand and may further comprise moisture, the process comprising the following steps:

   - calculating the amount (X1) of a macrocyclic ligand which is necessary for the batch size;
   - measuring the amount of moisture which is present in one or more samples of said batch;
   - calculating the total amount (M) of moisture which is present in the batch; and
   - calculating the total amount X3 = X1 + M of material which is required for preparing the batch size.

2. A process according to claim 1 wherein an amount (Y) of material comprising the macrocyclic ligand is added to the total amount X3 = X1 + M of material.

3. A process according to any of the claims 1 or 2 wherein the total amount (X3 = X1 + M) of material comprising the macrocyclic ligand and moisture is divided into a plurality of portions and packed into unit packages under controlled conditions of humidity.

4. A process according to any of the claims 1 to 3 wherein the macrocyclic ligand is DOTA, DO3A or HP-D03A.

5. A process, according to any of the claims 1 to 4 wherein the macrocyclic ligand is DOTA.

6. A process for producing a complex of a macrocyclic ligand with a lanthanide or similar compound, wherein a mixture of the material prepared according to any of the preceding claims and a lanthanide or similar compound is dissolved thereby obtaining said complex without a substantial amount of free lanthanide.

7. A process according to claim 6 wherein the lanthanide or similar compound is gadolinium, terbium or yttrium.

8. A process according to claim 7 wherein the lanthanide or similar compound is gadolinium.

9. A process for producing a liquid formulation comprising a macrocyclic ligand and a lanthanide or a similar compound, wherein:

   - the complex of a macrocyclic ligand with a lanthanide or similar compound is prepared according to any of the claims 6 to 8;
   - the amount of said macrocyclic ligand in a free form is present in the final formulation in an excess in the range from 0.02% to 0.4 mol% in relation to the total amount of the lanthanide.

10. A process according to claim 9 wherein the amount of free macrocyclic ligand is present in the final formulation in an excess in the range from 0.02% to 0.3 mol% in relation to the total amount of the lanthanide or similar compound.

11. A process according to claim 10 wherein the amount of free macrocyclic ligand is present in the final formulation in an excess in the range from 0.025% to 0.25 mol% in relation to the total amount of the lanthanide or similar compound.

12. A process according to claim 9 wherein excipients are added to the formulation, said excipients selected from water, an organic base, hydrochloric acid and/or sodium hydroxide for pH adjustment.

13. A process according to claim 12 wherein the hydrochloric acid and/or sodium hydroxide is added to obtain a pH in the range from 4 to 8.5.

14. A process according to claim 13 wherein the hydrochloric acid and/or sodium hydroxide is added to obtain a pH in the range from 6.5 to 7.9.

15. A process according to claim 12 wherein the organic base is meglumine and/or a salt thereof.

16. A process according to claim 9 wherein the lanthanide or similar compound is gadolinium, terbium or yttrium and wherein the macrocyclic ligand is DOTA, DO3A or HP-DO3A and the macrocyclic ligand is present in said liquid formulation in a concentration in the range from 0.3 to 0.7 M.

17. A process according to claim 16 wherein the macrocyclic ligand is present in said liquid formulation in a concentration in the range from 0.4 to 0.6 M.

Europäisches
Patentamt

European
Patent Office

Office européen
des brevets

# EUROPEAN SEARCH REPORT

Application Number

EP 13 16 2339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUKIS ET AL: "Optimized conditions for chelation of yttrium-90-DOTA immunoconjugates.", THE JOURNAL OF NUCLEAR MEDICINE, vol. 39, no. 12, 1 December 1998 (1998-12-01), pages 2105-2110, XP055039607, ISSN: 0161-5505 * page 2107 * * page 2108, left-hand column * | 1-17 | INV. A61K49/18 A61K49/10 |
| X | FR 2 927 539 A1 (GUERBET SA [FR]) 21 August 2009 (2009-08-21) * examples * * claims * | 1-17 | |
| X | DE 20 2008 010019 U1 (GUERBET SA [FR]) 2 July 2009 (2009-07-02) * example 3 * | 1-17 | |
| X | US 2009/208421 A1 (MEYER DOMINIQUE [FR] ET AL) 20 August 2009 (2009-08-20) * example 2; table 3 * | 1-17 | TECHNICAL FIELDS SEARCHED (IPC) |
| X | FR 2 921 271 A1 (GUERBET SA [FR]) 27 March 2009 (2009-03-27) * example * | 1-17 | A61K |
| X | WO 91/15243 A1 (COCKBAIN JULIAN R M [GB]; NYCOMED AS [NO]) 17 October 1991 (1991-10-17) * examples 4-5 * | 1-17 | |
| X | CN 102 659 702 A (WUHAN INST TECHNOLOGY; MIN JI) 12 September 2012 (2012-09-12) * examples * | 1-17 | |

-/--

The present search report has been drawn up for all claims

| Place of search | Date of completion of the search | Examiner |
|---|---|---|
| The Hague | 13 August 2013 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 13 16 2339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 1 130 189 A (UNIV WUHAN [CN]) 4 September 1996 (1996-09-04) * examples 2-3 * ----- | 1-17 | |

TECHNICAL FIELDS
SEARCHED        (IPC)

The present search report has been drawn up for all claims

| Place of search | Date of completion of the search | Examiner |
|---|---|---|
| The Hague | 13 August 2013 | Dullaart, Anwyn |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 13 16 2339

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2013

| Patent document cited in search report | | Publication date | Patent family member(s) | | Publication date |
|---|---|---|---|---|---|
| FR 2927539 | A1 | 21-08-2009 | NONE | | |
| DE 202008010019 | U1 | 02-07-2009 | NONE | | |
| US 2009208421 | A1 | 20-08-2009 | CA | 2714118 A1 | 27-08-2009 |
| | | | CN | 101977633 A | 16-02-2011 |
| | | | EP | 2242515 A2 | 27-10-2010 |
| | | | EP | 2591807 A1 | 15-05-2013 |
| | | | JP | 2011514334 A | 06-05-2011 |
| | | | KR | 20100133976 A | 22-12-2010 |
| | | | US | 2009208421 A1 | 20-08-2009 |
| | | | US | 2011129425 A1 | 02-06-2011 |
| | | | WO | 2009103744 A2 | 27-08-2009 |
| FR 2921271 | A1 | 27-03-2009 | FR | 2921271 A1 | 27-03-2009 |
| | | | WO | 2009037342 A2 | 26-03-2009 |
| WO 9115243 | A1 | 17-10-1991 | AT | 139449 T | 15-07-1996 |
| | | | AU | 655175 B2 | 08-12-1994 |
| | | | AU | 7565591 A | 30-10-1991 |
| | | | CA | 2079688 A1 | 03-10-1991 |
| | | | DE | 69120403 D1 | 25-07-1996 |
| | | | DE | 69120403 T2 | 31-10-1996 |
| | | | DK | 0523116 T3 | 15-07-1996 |
| | | | EP | 0523116 A1 | 20-01-1993 |
| | | | EP | 0727224 A2 | 21-08-1996 |
| | | | ES | 2088492 T3 | 16-08-1996 |
| | | | FI | 924396 A | 30-09-1992 |
| | | | GR | 3020563 T3 | 31-10-1996 |
| | | | HU | T62489 A | 28-05-1993 |
| | | | JP | H06507371 A | 25-08-1994 |
| | | | NO | 923821 A | 19-11-1992 |
| | | | RU | 2102082 C1 | 20-01-1998 |
| | | | US | 5384109 A | 24-01-1995 |
| | | | US | 5628983 A | 13-05-1997 |
| | | | US | 5738837 A | 14-04-1998 |
| | | | WO | 9115243 A1 | 17-10-1991 |
| CN 102659702 | A | 12-09-2012 | NONE | | |
| CN 1130189 | A | 04-09-1996 | NONE | | |

EPO FORM P0459

For more details about this annex : see Official Journal of the European Patent Office, No. 12/82

**EP 2 786 768 A1**

**Patent documents cited in the description**

- EP 0270483 A
- US 4647447 A
- WO 2010130814 A
- WO 2009103744 A